# EUROPEAN PATENT APPLICATION

(11) **EP 4 539 053 A1**
(43) Date of publication of application: **16.04.2025**
(21) Application number: 23290037.3
(22) Date of filing: 12.10.2023
(51) Int. Cl.: G16H 10/40, G16H 50/20, G01N 35/00

(54) **EXTRACTING LABORATORY, CLINICAL OR DIAGNOSTIC INFORMATION FROM A CLINICAL TEST**

(71) Applicant: Beckman Coulter, Inc., Brea, CA 92821 (US)
(72) Inventor: Denisselle, Benoit, 62400 Béthune (FR); Varlet, Eric, 59790 Ronchin (FR)
(74) Representative: Maiwald GmbH

(57) **Abstract**

Aspects relate to a method, a data processing system, a laboratory instrument, a computer program and a computer-readable medium. The method comprises obtaining, by a computing device, a relational result for a first clinical test. The first clinical test is carried out on a biological sample by a first laboratory instrument, the biological sample being in a sample container. The relational result comprises first information and second information, the first information specifying that a laboratory result for the first clinical test is outside a reliability range for the first clinical test carried out by the first laboratory instrument and the second information specifying one or more of an upper bound and a lower bound of the reliability range. The method further comprises assessing, by the computing device, whether a condition of a laboratory rule is determinable by using the first information and the second information. The laboratory rule specifies one or more actions that are to be taken depending on the outcome of a determination of the condition. At least one action of the one or more actions is to be taken on one or more of the biological sample, the sample container and the relational result and/or the one or more actions comprise displaying information about one or more of the biological sample, the sample container, the relational result and a patient. If the condition is determinable and, according to the outcome of the determination of the condition, the one or more actions are to be taken, the method comprises causing, by the computing device, the one or more actions to be taken.

## Description

The technical field is laboratory operations. More specifically, aspects relate to control and operation of a laboratory instrument (e.g., a laboratory analyzer).

Conventionally, when a clinical test is carried out on a biological sample by a laboratory instrument and a laboratory result for the clinical test is outside a reliability range of the clinical test carried out by the laboratory instrument, the laboratory result may be unusable or difficult to use In some cases, when the laboratory result is outside the reliability range, the laboratory instrument reports no result, or the laboratory result is incorrect. In addition or alternatively, there may be no way to further manipulate the laboratory result, e.g., because the numerical result provided by the analyzer is not reliable and cannot be used to determine whether the conditions of laboratory rules triggering some actions are fulfilled or not
Accordingly, after carrying out a clinical test on a biological sample by a laboratory instrument, it may be desirable to obtain a result for the clinical test that includes a numeric value that can be further manipulated.

According to a first aspect, a method is provided. The method comprises obtaining, by a computing device, a relational result for a first clinical test. The first clinical test is carried out on a biological sample by a first laboratory instrument, the biological sample being in a sample container. The relational result comprises first information and second information, the first information specifying that a laboratory result for the first clinical test is outside a reliability range for the first clinical test carried out by the first laboratory instrument and the second information specifying one or more of an upper bound (i.e., upper limit) and a lower bound (i.e., lower limit) of the reliability range. In particular, the second information specifies the upper bound (i.e., upper limit) or the lower bound (i.e., lower limit) of the reliability range.

The method further comprises assessing, by the computing device, whether a condition of a laboratory rule is determinable (i.e., determinate) by using the first information and the second information. The laboratory rule specifies one or more actions that are to be taken depending on the outcome of a determination of the condition. At least one action, e.g., each action, of the one or more actions is to be taken on one or more of the biological sample, the sample container and the relational result and/or the one or more actions comprise displaying information about one or more of the biological sample, the sample container, the relational result and a patient. For example, information about the patient may specify a condition or status of the patient associated with the biological sample. The condition or status of the patient may specify a disease or syndrome that affects the patient.

If the condition is determinable and, according to the outcome of the determination of the condition, the one or more actions are to be taken, the method comprises causing, by the computing device, the one or more actions to be taken.

Hence, the one or more actions that are caused to be taken may comprise:
- the at least one action of the one or more actions to be taken on one or more of the biological sample, the sample container and the relational result, and/or
- displaying information about one or more of the biological sample, the sample container, the relational result and a patient.

In particular, assessing whether the condition of the laboratory rule is determinable comprises comparing the relational result with a reference numerical value.

Obtaining the relational result for the first clinical test may be carried out after a first run of the first clinical test.

The biological sample may be associated with a patient. For example, the sample container may include a machine-readable code (e.g., a barcode) identifying the patient. Accordingly, a surgical procedure may be scheduled and can only take place after the laboratory result has been received and assessed (e.g., using the relational result). Moreover, a patient's life may depend on the laboratory result being assessed by a specified completion time, e.g., so that a life-saving surgical procedure may be performed.

A clinical test (e.g., the first clinical test) may be diagnostic and/or medical. The clinical test (i.e., clinical laboratory test) may include use of a chemical and/or biological process to determine or measure a level of a chemical component in the biological sample (e.g., bodily fluid and/or bodily tissue). The chemical component may include blood glucose, an electrolyte, an enzyme, a hormone, a lipid (fat), another metabolic substance, a protein or any other measurable analyte. The clinical test may be used to identify a sign of a nutrient deficiency in a patient, detect a change in the health of the patient, evaluate bodily functions of the patient (e.g., kidney function, liver function or thyroid function), monitor treatment of the patient or disease progression in the patient. In particular, the clinical test may comprise physical, biological, optical, mechanical, immunological, and/or chemical procedures. For example, the clinical test may be an immunoassay test. The biological sample may be a material or specimen and may include blood, urine, tissue, cells, saliva, and the like.

The method may be for controlling and/or operating the first laboratory instrument. The method may be for improving extraction of laboratory, clinical or diagnostic information from the clinical test. More particularly, the method may lead to an improvement in the extraction of laboratory, clinical or diagnostic information from the clinical test when carrying out the clinical test produces a laboratory result outside the reliability range for the first clinical test carried out by the first laboratory instrument.

Laboratory results of a laboratory instrument (e.g., the first laboratory instrument) may include measurements of the laboratory instrument.

For example, the laboratory result may be outside the reliability range for the first clinical test carried out by the first laboratory instrument when a concentration of a substance (i.e., an analyte) that the first clinical test is to measure in the biological sample is too high or too low to be measured (i.e., too high or too low to be measured accurately).

The first laboratory instrument may be an analyzer, i.e. a device used in a clinical laboratory to perform laboratory tests on biological samples. The laboratory instrument may be designed to measure clinical parameters, e.g., chemical substances, cellular components, or biomarkers. The laboratory instrument may be designed to assist in one or more of the diagnosis, monitoring and treatment of medical conditions. In particular, the first laboratory instrument may be comprised in an automated laboratory system (ALS).

The method may enable the laboratory rule to be tested with all available information regarding the laboratory result, thereby extending the usability of the laboratory result provided by the first laboratory instrument as much as possible and extending the applicability of workflows that include use of the first laboratory instrument.

For instance, an action of the one or more actions may include moving the sample container to an error region. In addition or alternatively, an action of the one or more actions may include flagging the relational result as unreliable and/or displaying the relational result. The actions do not include business or administrative activities, such as pricing or carrying out contract obligations. For instance, a laboratory rule may read as follows:
IF (*result of CSF glucose concentrations <= 18 mg*/*dL*) THEN (*flag the result* as *"High risk of bacterial meningitis"*),
wherein the condition is "result of CSF glucose concentration < 18 mg/dL" and the action is "flag the result as "High risk of bacterial meningitis". In this example, the action is taken if the condition is fulfilled (i.e., is true). For example a laboratory rule may read as follows:
(*flag the result* as *"High risk of bacterial meningitis"*) *EXCEPT IF* (*result of CSF glucose concentrations* ≥ *18 mg*/*dL*)
In this case, the condition is "result of CSF glucose concentrations > 18 mg/dL" and the action is "flag the result as "High risk of bacterial meningitis". In this case, the action is taken if the condition is not fulfilled (i.e. is false).

Causing the one or more actions to be taken may comprise providing instructions, e.g. to the first laboratory instrument or to another laboratory instrument, different from the first one, to carry out the one or more actions, initiating the one or more actions or carrying out the one or more actions.

A laboratory may be a technical system including one or more laboratory instruments. The laboratory may be a clinical or medical laboratory. The laboratory may include one or more laboratory instruments, e.g. an ALS comprising a plurality of analyzers. The laboratory may include at least one computer.

The laboratory may be configured to receive biological samples from clinical or medical facilities, such as hospitals. Alternatively, the laboratory may be a part of a clinical or medical facility and may receive biological samples from another part of the clinical or medical facility. The laboratory instruments may be capable of communicating with each other and/or communicating with other devices (e.g., computers). The laboratory instruments may also be referred to as laboratory devices or laboratory equipment and may include laboratory automation.

The first clinical test may be carried out in the laboratory.

Obtaining the relational result may comprise converting (i.e., transforming), by the first laboratory instrument, the laboratory result into the relational result. In particular, obtaining the relational result may comprise generating the relational result by using the laboratory result.

The computing device may be part of or connected to the first laboratory instrument. In addition or alternatively, the computing device may be communicatively coupled to the first laboratory instrument. In some cases, the computing device may be physically separate from the first laboratory instrument.

In particular, the condition is determinable by using the first information and the second information if the first and the second information allow for determining whether the condition is verifiable (i.e., provably true) or falsifiable (i.e., provably false) in correspondence to the laboratory result. In particular, the condition is determinable by using the first information and the second information if it is possible to establish, by using the first and second information, whether, in correspondence to the laboratory result, the condition is true or false. For instance, if the condition is of the type *(laboratory result < 100 ml*/*l*), and the first and the second information specify that the laboratory result is less than 50 ml/l, the condition is true in correspondence to the laboratory result and, hence, it is verifiable. For example, if the condition is of the type *(laboratory result > 100 ml*/*l*), and the first and the second information specify that the laboratory result is less than 50 ml/l, the condition is false in correspondence to the laboratory result and, hence, it is falsifiable. Exemplarily, if the condition is of the type (laboratory *result > 100 ml*/*l*), and the first and the second information specify that the laboratory result is less than 150 ml/I, the condition is not determinable, as it cannot be established whether the condition is true or false in correspondence to the laboratory result.

For instance, assessing, by the computing device, whether a condition of a laboratory rule is determinable comprises assessing whether the condition is true or false by using the first information and the second information.

In particular, the present invention allows for extracting more information from the first clinical test than would otherwise be possible, i.e, by checking whether the condition associated with the laboratory rule may be determined by using the information comprised in the relational information. In some cases, the relational result may make it possible to dispense with further clinical tests or to use a less capable laboratory instrument to carry out the first clinical test. Moreover, the relational result may make it possible to obtain information regarding the condition of the patient more quickly and/or to provide information regarding the condition of the patient to medical personnel sooner so that an action regarding the condition of the patient can be taken more quickly.

Exemplarily, if the condition is not determinable, the method may further comprise, causing, e.g. by the computing device, one or more other actions to be taken. For instance, the one or more other actions may comprise carrying out a second clinical test. In particular, the second clinical test may be more specific, accurate and/or may have a broader reliability range than the first clinical test. This way, the test that is more specific and/or more accurate and/or that has a broader reliability range is carried out only if and when actually needed, i.e., when the first clinical test does not allow for determining whether the condition is fulfilled, thereby improving the allocation of the resources of the laboratory and/or the ALS. Put another way, improving the extraction of information from the laboratory result of the first clinical test, the relational result makes it possible to forgo or dispense with additional clinical tests, thereby enabling ALS resources that would have been used for the additional clinical tests to be used for other purposes.

For example, the one or more other actions comprise diluting the biological sample and re-running the first clinical test in the diluted biological sample. For instance, the one or more other actions comprise notifying a user that the condition is not determinable and/or storing, in a computer-readable medium, information specifying that the condition is not determinable.

In particular, the one or more actions may comprise carrying out a third clinical test.

The first clinical test may differ from the second clinical test and/or the third clinical test. For example, the second clinical test and/or the third clinical test may be more specific than the first clinical test. In more detail, the first clinical test may relate to testing whether a concentration of substance is above or below a threshold and the second clinical test and/or the third clinical test may involve testing for a condition that only occurs if the concentration of the substance is above (or respectively below) the threshold. Accordingly, the second clinical test and/or the third clinical test may be more specific than the first clinical test in the sense that the second clinical test and/or the third clinical test are dependent on the first clinical test and/or the laboratory result for the first clinical test determines whether the second clinical test and/or the third clinical test are relevant. In particular, the second clinical test and the third clinical test may be equal to one another, i.e. the same clinical test.

In some cases, the second clinical test and/or the third clinical test may be a confirmatory test to confirm a diagnosis. In addition or alternatively, the second clinical test and/or the third clinical test may be directed to determining a condition or status of a patient associated with the biological sample. For example, the biological sample may include a bodily fluid (e.g., blood or urine) from the patient. Determining the condition or status of the patient may comprise determining whether the patient has a disease or syndrome.

A testing methodology may be based on established scientific principles, possibly involving biology, chemistry, and/or physics. The testing methodology may define a process (i.e., procedure) including one or more steps that is used to test biological samples for one or more analytes. One or more steps of the testing methodology may involve the use of one or more reagents. Different methodologies may be used to test for the same analyte. For example, the testing methodology may be used to test for the presence of an antigen in the biological sample. The reaction of the antigen that is present in the biological sample to a first antibody is compared with reactions of known concentrations and the amount of the antigen is reported. In a different testing methodology, a different antibody may be used to test for the same antigen. As another example, biological samples having different degrees of endogenous interferences of hemoglobin, bilirubin and lipids may be used in different methodologies. For example, the second testing methodology may differ from the first testing methodology in that the former methodology uses a different reagent than the reagent used in the latter methodology.

The first clinical test may comprise measuring a first clinical parameter using a first testing methodology. In particular, the first clinical test is configured to measure the first clinical parameter using the first testing methodology. The second clinical test may comprise measuring the first clinical parameter using a second testing methodology. -In particular, the second clinical test is configured to measure the first clinical parameter using the second testing methodology. The first testing methodology may differ from the second testing methodology. The third clinical test may comprise measuring the first clinical parameter using a third testing methodology. -In particular, the third clinical test is configured to measure the first clinical parameter using the third testing methodology. The first testing methodology may differ from the third testing methodology.

The first clinical test may comprise measuring a first clinical parameter (e.g., a first analyte), the second clinical test may comprise measuring a second clinical parameter (e.g., a second analyte) and/or the third clinical test may comprise measuring a third clinical parameter (e.g., a third analyte). The first clinical parameter may differ from the second clinical parameter and/or from the third clinical parameter.

Specifically, the clinical test (e.g., the first clinical test) may be a glucose test, e.g., a test to determine (e.g., measure) the concentration of glucose in the biological sample.

More specifically, the first clinical test may be a fasting plasma glucose test or a random plasma glucose test. Accordingly, the second clinical test and/or third clinical test may relate to confirmation, further evaluation or type classification based on the first clinical test. For example, the second clinical test and/or third clinical test may be a test to confirm diabetes or confirm a specific type of diabetes, e.g., the second clinical test and/or third clinical test may be an oral glucose tolerance test or a hemoglobin A1 c test to confirm diabetes Mellitus. Alternatively, the second clinical test and/or third clinical test may confirm hypoglycemia based on a low blood glucose level determined in the first clinical test. Accordingly, the second clinical test and/or third clinical test may comprise a glucose tolerance test or continuous glucose monitoring to evaluate glucose levels over a period of time.

In another example, the first clinical test may be a glucose challenge test. Abnormal results of the glucose challenge test may indicate gestational diabetes. Accordingly, the second clinical test and/or third clinical test may confirm a diagnosis of gestational diabetes, e.g., the second clinical test and/or third clinical test may be the oral glucose tolerance test. The second clinical test and/or third clinical test may be used for diagnosis and/or to determine appropriate management of a diagnosed condition.

In yet another example, the first clinical test may be the fasting plasma glucose test and may indicate elevated glucose levels. Accordingly, the second clinical test and/or third clinical test may be used to confirm Cushing's syndrome. Specifically, the second clinical test and/or third clinical test may be a dexamethasone suppression test or a 24-hour urinary free cortisol test to assess cortisol levels.

As a further example, the first clinical test may be the fasting plasma glucose test. Abnormal results may suggest pancreatic dysfunction. Accordingly, the second clinical test and/or third clinical test may be used to test lipase and/or amylase levels, perform pancreatic imaging or perform endoscopic procedures, such as endoscopic ultrasound or endoscopic retrograde cholangiopancreatography for further evaluation.

The reliability range for a clinical test (e.g. the first clinical test) carried out by a laboratory instrument (e.g. the first laboratory instrument) may be referred to as an analytic measuring range. In particular, the analytic measuring range is typically provided by the manufacturer of that laboratory instrument. In particular, the reliability range for a clinical test (e.g. the first clinical test) carried out by a laboratory instrument (e.g. the first laboratory instrument) may be a range within which that clinical test can accurately and/or precisely measure a corresponding clinical parameter with one or more of: an accuracy within a given accuracy range, a precision within a given precision range, a specificity within a given specificity range, and a sensitivity within a given sensitivity range. The reliability range for a clinical test carried out by a laboratory instrument may specify the lowest concentration and/or the highest concentration of an analyte that the clinical test carried out by the laboratory instrument can detect and quantify with one or more of: an accuracy within a given accuracy range, a precision within a given precision range, a specificity within a given specificity range, and a sensitivity within a given sensitivity range.

Precision (i.e., the precision component of the reliability range) may measure the consistency of laboratory results when the same biological sample is repeatedly tested. In particular, within-run precision measures the consistency of laboratory results when the same biological sample is repeatedly tested within the same day. In particular, within-lab precision measures the consistency of laboratory results when the same biological sample is repeatedly tested over multiple days, e.g., for a specified number of day set for an experiment. For example, multiple biological samples may be evaluated for precision or imprecision. For each set of clinical tests, two biological samples may be chosen, one with low concentration of interferent (e.g., about 0.3-0.5 g/L hemoglobin for hemolysis or about 50-100 µmol/L bilirubin for icteria) and one with high concentration of interferent (e.g., about 1.0-2.0 g/L hemoglobin for hemolysis, at least about 300 µmol/L bilirubin for icteria). In some cases, the biological samples may be analyzed in duplicate, two times per day over 5 days, for a total of about 20 measurements for each sample. The results may be compared to a known guideline, e.g., the Clinical and Laboratory Standards Institute (CLSI) EP5-A2 guideline or the National Committee for Clinical Laboratory Standards (NCCLS) EP5-A guideline.

Precision may be expressed as a coefficient of variation (CV) and may indicate a variability between replicated laboratory results. A lower CV may indicate a higher precision and/or a higher reliability. In other words, CV may be inversely proportional to precision and/or reliability. For example, a CV of less than 3% may be considered acceptable for the first laboratory instrument, a second laboratory instrument and/or a third laboratory instrument.

Accuracy may refer to the difference between a laboratory result provided by a laboratory instrument and a reference (i.e., known or true) value. Accuracy may be assessed by comparing laboratory results from a laboratory instrument to a known standard. Accuracy may be reported as a bias percentage or as a percentage of laboratory results falling within a specified range of the reference value.

Specificity may measure a capability of the laboratory instrument to accurately identify and/or measure a desired analyte without interference from other substances. Specificity may indicate the degree to which laboratory results produced by a laboratory instrument are affected by cross-reactivity or interference from other analytes or substances present in the biological sample.

Sensitivity may refer to a capability of the laboratory instrument to detect low concentrations of an analyte. Sensitivity may be evaluated by determining the lowest concentration of the analyte that can be reliably detected and/or distinguished from 0 (e.g., with a probability of at least about 95%) and quantified by the laboratory instrument. For example, sensitivity limits may be determined in accordance with a guideline, e.g., the CLSI EP17-A2.

The reliability range may depend on the biological sample being tested. For example, a laboratory test for a concentration of an analyte in serum may have a different reliability range than a laboratory test for the concentration of an analyte in urine or cerebrospinal fluid (CSF).

Potassium (K) measurements may be used in the diagnosis of hypokalemia (metabolic alkalosis, metabolic acidosis or the absence of acid-base disturbances), hyperkalemia (over-administration of potassium, acidosis, or crush injuries), renal failure, Addison's disease or other diseases involving an electrolyte imbalance. For example, the reliability ranges for a potassium (K) test carried out by a first exemplary chemistry analyzer may be (mmol/L = millimoles per liter):
∘ 1.0 - 15.0 mmol/L when the biological sample is serum or plasma; and
∘ 2 - 300 mmol/L when the biological sample is urine.

In particular, the values included in the reliability ranges defined above have a sensitivity greater than 95%. Hence, the given sensitivity range is (95%, 100%). For instance, the values included in the reliability ranges defined above have a within-run precision with a relative CV below 2.0% when the biological sample is serum or plasma and below 4.0% when the biological sample is urine. Hence, in this case, when the biological sample is serum or plasma the given within-run precision range may be represented by the given relative CV range (0, 2.0%). When the biological sample is urine, the given within-run precision range may be represented by the given relative CV range (0, 4.0%).

For example, the reliability ranges for a glucose test carried out by a second exemplary chemistry analyzer may be (mg/dL = milligrams per deciliter):
∘ 10 - 810 mg/dL when the biological sample is serum, plasma or CSF; and
∘ 3.6 - 810 mg/dL when the biological sample is urine.
In particular, the values included in the reliability ranges defined above have a sensitivity greater than 95%. Hence, the given sensitivity range is (95%, 100%). For instance, the values included in the reliability ranges defined above have a within-run precision with a relative CV ≤3.0% irrespective of whether the biological sample is serum, plasma CSF or urine. Hence, in this case, the given within-run precision range may be represented by the given relative CV range (0, 3.0%]. The values included in the reliability ranges defined above have a within-laboratory precision with a relative CV ≤ 3.0% irrespective of whether the biological sample is serum, plasma CSF or urine. Hence, in this case, the given within-laboratory precision range may be represented by the given relative CV range (0, 3.0%].

Hence, an accurate laboratory result of the glucose test carried out by the second exemplary laboratory instrument on serum, plasma, or cerebrospinal fluid is comprised in the range between 10 and 810 mg/dL. Similarly, an accurate laboratory result of the glucose test carried out by the second exemplary laboratory instrument on urine is comprised in the range between 3.6 and 810 mg/dL. A laboratory result for the concentration of an analyte in a biological sample of serum/plasma/CSF < 10 or > 819 mg/dL would be outside the reliability range of the laboratory instrument. Similarly, a laboratory result for the concentration of an analyte in a biological sample of urine < 3.6 or > 810 mg/dL would be outside the reliability range of the laboratory instrument.

In the context of the reliability range for the glucose test carried out by the second exemplary laboratory instrument, if the laboratory result is below 10 mg/dL (e.g., the laboratory result is 5 mg/dL) then the relational result may be <, 10 mg/dL. Continuing the example, if the condition of the following laboratory rule were assessed, the computing device would cause an action to be taken:
*IF (result of CSF glucose concentrations <= 18 mg*/*dL) THEN (flag the result* as *"High risk of bacterial meningitis")*
This is because the relational result of <, 10 mg/dL means that the condition of the laboratory rule above is determinable and TRUE.

For example, the reliability range for an Immunoglobulin M test carried out by a third exemplary chemistry analyzer on serum or plasma is 0.2 - 5.0 g/L (g/L = grams per liter). For instance, the reliability range for a Prealbumin test carried out by a third exemplary chemistry analyzer on serum is 0.03 - 0.8 g/L.

Causing the one or more other actions to be taken may comprise causing a second laboratory instrument to carry out the second clinical test. Alternatively or additionally, causing the one or more actions to be taken may comprise causing a third laboratory instrument to carry out the third clinical test.
The second laboratory instrument and/or the third laboratory instrument may be configured to measure the first parameter with a higher sensitivity, precision, specificity, linearity, accuracy than the first laboratory instrument. In particular, the second laboratory instrument and/or the third laboratory instrument may have a reliability range for the first parameter that encompasses the reliability range for the first parameter of the first laboratory instrument or a reliability range for the first parameter that includes the laboratory result. Accordingly, the laboratory instruments may have reliability ranges for the first parameter such that a laboratory result is outside a reliability range of the first laboratory instrument but inside the reliability range of the second laboratory instrument and/or the third laboratory instrument. In addition or alternatively, the laboratory result might not be determinable for the first laboratory instrument but may be determinable for the second laboratory instrument and/or the third laboratory instrument, e.g., because the first parameter may be measured with higher sensitivity by the second laboratory instrument and/or the third laboratory instrument than by the first laboratory instrument.

A laboratory instrument (e.g., the first laboratory instrument, the second laboratory instrument and/or the third laboratory instrument) may be a laboratory analyzer, e.g. comprised in an ALS. In particular, the first laboratory instrument and one or more of the second laboratory instrument and the third laboratory instrument are comprised in the ALS.

The laboratory instrument may be a medical or clinical analyzer. The laboratory instrument may also include one or more pre-analysis, analysis and post-analysis components.

An analyzer is in particular an instrument configured to carry out one or more analytic steps, such as measuring one or more characteristics of the sample, e.g. the concentration of a given analyte. The analysis laboratory instrument may be or include an immunoassay analyzer, a chemistry analyzer, an identification and antibiotic susceptibility analyzer, a bacteriology analyzer, a molecular analyzer, a hematology analyzer or a urinalysis instrument.

An automation component may be for moving containers from one laboratory instrument to another laboratory instrument or for moving containers from one component of a laboratory instrument to another component of the same laboratory instrument or to another component of another laboratory instrument. The automation component may include a track, a belt and/or a tube carrier configured to move biological samples.

Generally, an Automated Laboratory System (herein also referred to as: "ALS") is an assembly comprising a plurality of components and a computing device, wherein the computing device is operatively connected to these components and is configured to control each component. A component may be an analyzer, a pre-analysis laboratory instrument, a post-analysis laboratory instrument, an input/output module, an automation component (e.g. track, belt, tube carrier) configured to move a sample. In particular, an ALS may comprise one or more subsystems, wherein a subsystem comprises one or more components of the ALS.

A pre-analysis laboratory instrument is an instrument configured to carry out one or more pre-analytic steps on a biological sample to prepare said sample for the analytic instrument(s). A pre-analysis laboratory instrument may include a centrifuge, a decapper, a recapper and/or an aliquoter.

A post-analysis instrument is an instrument configured to carry out one or more post-analytic steps on the biological sample after the sample has been processed by one or more analytic instruments. A post-analysis laboratory instrument may include a recapper (e.g., for putting caps back on containers), a storage device, a refrigerator or an input/output device. Both pre-analysis and post-analysis laboratory instruments may be referred to as peri-analytical laboratory instruments.

In one example, a hematology and a urinalysis test may be carried out on a female patient based on symptoms including a complaint of pain on her right side, a temperature of 37.5°C, a pulse of 103, blood pressure 150/80, and a respiratory rate of 19. The laboratory workflow may include a first laboratory rule that specifies an action to be taken, if a condition depending on the results of the hematology and the urinalysis test is met. For example, the first laboratory rule may read as follows:
IF (*WBC₁* > *T_{WBC1} AND MDW > T_{MDW}*) AND (WBC₂ > T_{WBC2} AND BC > T_{BC}) THEN (*carry out a chemistry* & *immunoassay test*)*,*
wherein WBC₁ refers to a white blood cell count from the hematology test and MDW refers to the Monocyte Distribution Width from the hematology test. The urinalysis test may measure the bacterial count (BC) and a new value of the white blood count (WBC₂). wherein T_{WBC1}, T_{WBC2} and T_{MDW} are two thresholds for the white blood count and one threshold for Monocyte Distribution Width, respectively. T_{BC} is a threshold for the bacterial count. The action of carrying out the chemistry and immunoassay test is taken if the condition (*WBC₁* > *T_{WBC1} AND MDW > T_{MDW}*) AND (WBC₂ > T_{WBC2} AND BC > T_{BC}) is met. If, for example, the two aforementioned white blood cell counts, the aforementioned monocyte distribution width and the bacterial count fall outside their respective reliability range, the action is taken if, e.g.:
- The relational result associated with the white blood cell count from the hematology test is of the type >, U_{WBC1}, wherein U_{WBC1} is a numerical value greater than T_{WBC1}; and
- The relational result associated with the Monocyte Distribution Width from the hematology test is of the type >, U_{MDW}, wherein U_{MDW} is a numerical value greater than T_{MDW}; and
- The relational result associated with the white blood cell count from the urinalysis test is of the type >, U_{WBC2}, wherein U_{WBC2} is a numerical value greater than T_{WBC2}; and
- The relational result associated with the bacterial count from the urinalysis test is of the type >, U_{BC}, wherein U_{BC} is a numerical value greater than T_{BC}.
In this case, the chemistry and immunoassay test carried out on a third laboratory instrument may indicate elevated lactic acid, elevated C-Reactive Protein (CRP), elevated Procalcitonin (PCT), and elevated interleukin (IL-6). These elevated values may cause the condition of a laboratory rule to be met and, hence, one or more actions specified by the third laboratory rule to be taken. For instance, the one or more actions may comprise (i) displaying the laboratory results and the implications of the relational results obtained by carrying out the hematology test, the urinalysis test and the chemistry and immunoassay test and/or (ii) transmitting an alert. In this example, the one or more actions may lead to a diagnosis of E.coli, for which the female patient can be treated.

The one or more actions may comprise rerunning (i.e., repeating or reiterating) the first clinical test. In other words, the one or more actions may comprise carrying out the first clinical test a second time. In addition or alternatively, rerunning the first clinical test may be carried out after diluting or otherwise modifying the biological sample.

For example, the laboratory rule may be as follows:
*IF (result > upper limit of reliability range) THEN rerun with dilution*

In the example above, the condition is *"result > upper limit of reliability range"* and the action to be taken is *"rerun with dilution".*

Another example of a laboratory rule is as follows:
*IF (result of CSF glucose concentrations <= 18 mg*/*dL) THEN (flag the result* as *"High risk of bacterial meningitis"*)
Hence, if the relational result indicates that the glucose concentration in a biological sample of CSF is less than or equal to 18 mg/dL (1.0 mmol/L) then an action may be caused to be taken, e.g., the laboratory result corresponding to (e.g., specified by) the relational result may be flagged as indicative of bacterial meningitis, a corresponding alert may be displayed, the first information and/or the second information may be stored by or transmitted to a computer, the first clinical test may be rerun or the second third clinical test to confirm the result may be carried out.

A reagent may be a substance or compound that can facilitate a chemical reaction. The reagent may include one or more of the following: a solvent (e.g., water), an enzyme, a catalyst. The reagent may have one of various types known for use in analysis of biological specimens. Some examples of reagents include liquid reagents containing labeled specific-binding reagents, for example antibody or nucleic acid probes, liquid reagents containing reactive and/or non-reactive substances, red blood cells suspensions, and particle suspensions. In other examples, the reagent can be a chemiluminescent substrate. The reagent may be an assay reagent. The assay reagent may include wash buffers, rinses, sample pretreatments, diluents, stains, dyes, substrates, antibody conjugates, enzymes or enzyme conjugates, nucleic acid conjugates, cell lysis reagents, and the like, in reacted or unreacted states. Components of assay reagents typically include water, buffers, chemicals, particles, substrates, enzymes, fixatives, preservatives, nucleic acids, antibodies, acids, bases, and mixtures thereof, in reacted or unreacted states.

The one or more actions may comprise diluting the biological sample. Diluting the biological sample may be carried out before rerunning the first clinical test. Diluting the biological sample may be carried out by adding one or more diluents to the biological sample. The diluents may include water and/or saline.

The one or more actions may comprise displaying the first information and/or the second information.

The one or more actions may comprise displaying third information. The third information may specify whether, based on the first information and the second information, the laboratory result may belong to one or more of a critical range, a warning range and a normal range. The critical range, the normal range and the warning range may be associated with a potential condition of the patient. In particular, a critical range is the range of values for the laboratory result that indicate a life-threatening condition e.g. for individuals that belongs to a given population. A normal range is in particular the range of values for the laboratory result that are considered normal e.g. among the members of a given population. A warning range is in particular the range of laboratory result that are neither normal nor life-threatening e.g. for individuals of a given population. In particular, a population is a set of individuals grouped by one or more of age, gender, ethnicity, geographical origin and the like.

Displaying the first information, the second information and/or the third information may have one or more of the following effects:
- to enable use of an otherwise unusable laboratory result;
- to enable a diagnosis to be carried out more quickly;
- to reduce the number of clinical tests needed.

These effects are achieved by displaying the relational result and/or the third information when the first laboratory result is outside the reliability range. Such laboratory results are conventionally discarded or displayed without context. By means of this displayed relational result or third information, even an unreliable laboratory result can be used, at least to certain extent, to enable diagnosis of a patient to be reached more quickly. In some cases, further clinical tests using more reliable laboratory instruments can be dispensed with.

In some cases, the second information specifies one or more of a unit of measure (hereinafter also referred to as: "first unit of measure") of the upper bound of the reliability range and a unit of measure (hereinafter also referred to as: "second unit of measure") of the lower bound of the reliability range. The inclusion of the information specifying the first unit of measure and/or the second unit of measure allows for reducing errors in the determination of the condition of the laboratory rule that may arise by comparing values expressed in different unit of measures.

Exemplarily, as disclosed above, assessing whether the condition of the laboratory rule is determinable comprises comparing the relational result, e.g. the upper bound and/or the lower bound specified in the second information, with a reference numerical value. In particular, the reference numerical value may be expressed in a third unit of measure.

If the second information specifies the first unit of measure, assessing whether the condition of the laboratory rule is determinable may comprise assessing whether the first unit of measure is equal to the third unit of measure. If the first unit of measure and the third unit of measure are different from one another, assessing whether the condition of the laboratory rule is determinable may comprise expressing the relational result and/or the reference numerical value in a first common unit of measure before comparing the relational result with the reference numerical value. More particularly, if the first common unit of measure is the third unit of measure, only the relational result is expressed in the first common unit of measure. If, instead, the first common unit of measure is the first unit of measure, only the reference numerical value is expressed in the first common unit of measure. In general, if the first common unit of measure is different from the first and the third unit of measure, both the relational result and the reference numerical value are expressed in the first common unit of measure.

If the second information specifies the second unit of measure, assessing whether the condition of the laboratory rule is determinable may comprise assessing whether the second unit of measure is equal to the third unit of measure. If the second unit of measure and the third unit of measure are different from one another, assessing whether the condition of the laboratory rule is determinable may comprise expressing the relational result and/or the reference numerical value in a second common unit of measure before comparing the relational result with the reference numerical value. More particularly, if the second common unit of measure is the third unit of measure, only the relational result is expressed in the second common unit of measure. If, instead, the second common unit of measure is the second unit of measure, only the reference numerical value is expressed in the second common unit of measure. In general, if the first common unit of measure is different from the second and the third unit of measure, both the relational result and the reference numerical value are expressed in the second common unit of measure.

Obtaining the relational result may comprise constructing, by using the laboratory result and one or more of the upper bound and the lower bound, the relational result. In particular, the step of constructing the relational result is carried out only if the laboratory result is outside the reliability range, e.g. greater than the upper bound or less than the lower bound.

Exemplarily, constructing the relational result may comprise comparing the laboratory result with the upper bound and the lower bound. If the laboratory result is greater than the upper bound or less than the lower bound, the method comprises generating the first information and the second information. If the laboratory result is comprised in the reliability range.

For instance, if the laboratory result is greater than the upper bound, the first information specifies that the laboratory result is greater than the upper bound and, hence, outside the reliability range. In this case, the first information may be specified by using the character ">", the string "greater than", the string "above", or the like. In particular, if the laboratory result is greater than the upper bound, the second information comprises the numerical value of the upper bound and/or a string specifying the upper bound.

Exemplarily, if the laboratory result is less than the lower bound, the first information specifies that the laboratory result is less than the lower bound and, hence, outside the reliability range. In this case, the first information may be specified by using the character "<", the string "less than", the string "below", or the like. In particular, if the laboratory result is less than the lower bound, the second information comprises the numerical value of the lower bound and/or a string specifying the lower bound.

Obtaining the relational result may comprise receiving the relational result, e.g., from the first laboratory instrument.

Assessing whether the condition of the laboratory rule is determinable may comprise determining, by using the relational result, a range to which the laboratory result belongs. The range to which the laboratory result belongs may be either a first range or a second range. The first range may consist of the values above the upper limit of the reliability range. The second range may consist of the values below the lower limit of the reliability range.

Assessing whether the condition of the laboratory rule is determinable may further comprise determining whether one of the first requirement and the second requirement is fulfilled. The first requirement may be the requirement that the condition is fulfilled for each value of the range to which the laboratory result belongs in the second requirement may be the requirement that the condition is not fulfilled for each value of the range to which the laboratory result belongs. The condition is determinable if one of the first requirement on the second requirement is fulfilled. In particular, the first range is the interval (upper bound, ∞), wherein the upper bound belongs to the first range only if the upper bound does not belong to the reliability range. Exemplarily, the second range is the interval (-∞, lower bound), wherein the lower bound belongs to the second range only if the lower bound does not belong to the reliability range.

In particular, the first requirement is fulfilled if the range to which the laboratory result belongs is included in the validity range of the condition. For instance, the second requirement is fulfilled if the validity range of the condition and the range to which the laboratory result belongs are disjoint from one another. The validity range of the condition is in particular, the range of values in correspondence of which the condition is fulfilled.

In some cases, the relational result may comprise one or more of a numerical value, a relational operator (e.g., =, >, <), and a unit of measure (e.g., a unit of measure of concentration, such as milliliters per liter, i.e., ml/L). Examples of relational results include the following:
1. >, *8000, µL*/*L* (first relational result);
2. >, *12000, µL*/*L* (second relational result);
3. <, *8000, µL*/*L* (third relational result); and
4. <, *12000, µL*/*L* (fourth relational result).

The numbered examples above are relational results including a relational operator followed by a numerical value followed by a unit of measure; the relational operator is separated from the numerical value by a comma and the numerical value is separated from the unit of measure by a comma. In the first example, the ">" relational operator is followed by the numerical value "8000" and the unit of measure microliters per liter (i.e., "µL/L").

A first exemplary laboratory rule corresponding to the numbered examples above may be the following:
*IF (result > 10 mlll) THEN (flag a specified pathology).*

The first exemplary laboratory rule may be used to test for the presence of the specified pathology based on the condition that the relational result is greater than 10 ml/l. In the laboratory rule above the condition is *"result > 10 ml*/*l"* and the action is "flag a specified pathology". The condition comprises a reference numerical value, i.e. 10 ml/l. The first exemplary laboratory rule may correspond to the first requirement, since the action is carried out when the condition is fulfilled (i.e., evaluates to true) for each value of the range to which the laboratory result belongs.

Assessing whether the condition of the first exemplary laboratory rule is determinable by using the first relational result comprises converting the first relational result in the unit of measure of the reference numerical value. After the conversion, the first relational result reads >, 8, ml/L. Hence, in this case, the range to which the laboratory result belongs is R1 = (8 ml/L, ∞). The validity range of the condition of the first exemplary rule is R2 = (10 ml/L, ∞). The range R1 is not comprised in the range R2 and R1 and R2 are not disjoint from one another. Hence, in this case, the condition is not determinable. In particular, in this case, the action specified in the primary laboratory rule is not taken.

Assessing whether the condition of the first exemplary laboratory rule is determinable by using the second relational result comprises converting the second relational result in the unit of measure of the reference numerical value. After the conversion, said relational result reads >, 12, ml/L. Hence, in this case, the range to which the laboratory result belongs is R3 = (12 ml/L, ∞). The range R3 is comprised in the range R2. Hence, in this case, the condition is determinable. In particular, the condition is true in correspondence to the laboratory result and hence, according to the first exemplary result, the action shall be taken.

Assessing whether the condition of the first exemplary laboratory rule is determinable by using the third relational result comprises converting the third relational result in the unit of measure of the reference numerical value. After the conversion, said relational result reads <, 8, ml/L. Hence, in this case, the range to which the laboratory result belongs is R4 = (-∞, 8 ml/L). The range R4 and the range R2 are disjoint from one another. Hence, in this case, the condition is determinable. In particular, the condition is false in correspondence to the laboratory result and, according to the first exemplary rule, the action shall not be taken.

Assessing whether the condition of the first exemplary laboratory rule is determinable by using the fourth relational result comprises converting the fourth relational result in the unit of measure of the reference numerical value. After the conversion, said relational result reads <, 12, ml/L. Hence, in this case, the range to which the laboratory result belongs is R5 = (-∞, 12 ml/L). The range R5 is not comprised in the range R2 and R5 and R2 are not disjoint from one another. Hence, in this case, the condition is not determinable. In particular, in this case, the action specified in the primary laboratory rule is not taken.

A second exemplary laboratory rule is as follows:
*(flag a certain pathology suspicion) EXCEPT IF (result < 10 mIll).*

The second exemplary laboratory rule includes an action of "flag a certain pathology condition" and a condition of "result < 10 ml/l". The condition of "result < 10 ml/l" of the second exemplary laboratory rule above may be referred to as an "exception condition" in view of the "EXCEPT IF" expression.

For the first relational result, the exception condition of the second exemplary laboratory rule is not determinable. Therefore, the action of flagging a certain pathology suspicion (i.e., the flagging action) will not take place. For the second relational result, the exception condition is not fulfilled and the flagging action is taken. For the third relational result, the exception condition is fulfilled and the flagging action is not taken. For the fourth relational result, the exception condition of the second exemplary laboratory rule is not determinable, then the flagging action will not take place.

A third exemplary laboratory rule is as follows:
*IF (result < 100 mlll) THEN (result is automatically accepted)*
Hence, if the condition, "result < 100 ml/l" is determinable and the outcome of the determination of the condition is TRUE (e.g., 1 rather than 0) then the computing device may cause the one or more actions to be taken, wherein the one or more actions include automatically accepting the laboratory result.

Exemplarily, if the condition is not determinable, the method further comprises one or more of the following:
- notifying a user that the condition is not determinable,
- storing, in a computer readable medium, information specifying that the condition is not determinable.

If the condition is not determinable, it may be that the laboratory rule is not applied.

In addition or alternatively, if the condition is not determinable, then the first clinical test may be carried out using a different methodology on a second laboratory instrument. Alternatively, if the condition is not determinable, then the first clinical test may be carried out using a different methodology on the first laboratory instrument.

In some examples, causing the one or more actions to be taken comprises initiating the taking of the one or more actions. Initiating the taking of the one or more actions may comprise sending instructions to the laboratory instrument, the instructions specifying that the one or more actions shall be taken. In particular, the laboratory instrument is configured to receive the instructions, access the information included in the instructions and follow the instructions, thereby taking the one or more actions.

For instance, causing the one or more actions to be taken comprises providing laboratory instrument with instructions to take the one or more actions. Exemplarily, the computer device may generate the instructions for one or more instrument components of the laboratory instruments. For instance, the instructions specify that the one or more actions shall be taken and the one or more instrument components are configured to receive the instructions, access the information included in the instructions and follow the instructions thereby taking the one or more actions.

In particular, causing the one or more actions to be taken comprises taking, e.g. by the laboratory instrument, the one or more actions.

The method may further comprise receiving the biological sample.

The method may further comprise carrying out the first clinical test.

According to a second aspect, a data processing system is provided. The data processing system comprises means for carrying out a method as described above. The data processing system may be included in a laboratory analyzer or in an automated laboratory system.

According to a third aspect, a laboratory instrument comprising the data processing system is provided. The laboratory instrument may be configured to receive the biological sample and/or to carry out the first clinical test.

According to a fourth aspect, a computer program is provided. The computer program may be implemented as part of a computer program product. The computer program comprises instructions which, when the program is executed by a computer, cause the computer to carry out a method as described above.

According to a fifth aspect, a computer readable medium is provided. The computer readable medium comprises instructions which, when executed by a computer, cause the computer to carry out a method as described above.

The subject matter described in this disclosure can be implemented as a method or on a device, possibly in the form of one or more computer programs (e.g., computer program products). Such computer programs may cause a data processing apparatus to perform one or more operations described in the present disclosure.

The subject matter described in the present disclosure can be implemented in a data signal or on a machine readable medium, where the medium is embodied in one or more information carriers, such as a CD-ROM, a DVD-ROM, a semiconductor memory, or a hard disk. In particular, disclosed subject matter may be tangibly embodied in a non-transitory machine (i.e., computer) readable medium.

In addition, the subject matter described in the present disclosure can be implemented as a system including a processor, and a memory coupled to the processor. The memory may encode one or more programs to cause the processor to perform one or more of the methods described in the application. Further subject matter described in the present disclosure can be implemented using various machines.

Details of one or more implementations are set forth in the exemplary drawings and description that follow. Other features will be apparent from the description, the drawings, and from the claims.

### Brief Description of the Figures

Figure 1 shows a computer 100 and an automated laboratory system (ALS) 200 that may be used in some embodiments.
Figure 2 shows a flow chart including steps of a method according to some embodiments.
Figure 3 shows an example of a display based on the laboratory result for a first clinical test and a reliability range for the first clinical test carried out by a laboratory instrument.
Figure 4 shows another example of the display.
Figure 5 shows yet another example of the display.
Figure 6 shows yet another example of the display.
Figure 7 shows yet another example of the display.
Figure 8 shows yet another example of the display.
Figure 9 shows yet another example of the display.
Figure 10 shows yet another example of the display.

### Detailed Description

In the following text, a detailed description of examples will be given with reference to the drawings. Various modifications to the examples may be made. In particular, one or more elements of one example may be combined and used in other examples to form new examples.

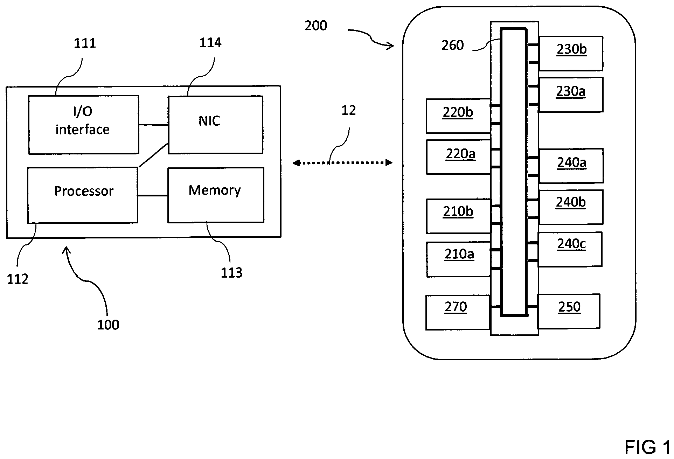

**Figure 1** shows a computer 100 and an automated laboratory system (ALS) 200 that may be used in some embodiments. The computer 100 and the automated laboratory system 200 may be part of a laboratory. The computer 100 may include an input/output (I/O) interface 111, a processor 112, a memory 113, and a network interface controller 114. One or more peripheral (input or output) devices may be connectable to the input/output interface 111. The peripheral input devices may include one or more of the following: a computer keyboard and/or a pointing device, such as a mouse. The peripheral output devices may include one or more of the following: a terminal, a printer, a disk drive or other storage device, and a video monitor. The processor 112 may include a central processing unit (CPU) and/or a graphics processing unit (GPU), each having one or more processing cores. The memory 113 may include primary memory, such as random access memory (RAM), read-only memory (ROM) or flash memory, and/or secondary memory, such as a solid-state drive or a hard disk drive.

The network interface controller 114 may be connectable to a computer network 12. The ALS 200 may also be connectable to the computer network 12, In some cases, the computer 100 may be included in (i.e., may be part of) the ALS 200. The ALS 200 may also be referred to as a laboratory instrument or an automated laboratory instrument. The ALS 200 may include immunoassay laboratory instruments 210a, 210b as well as 220a and 220b. For instance, the immunoassay laboratory instruments 210a and 210b are immunoassay instruments of a first type and the immunoassay laboratory instruments 210a and 210b are immunoassay instruments of a second type. Exemplarily, the immunoassay laboratory instruments 210a and 210b may have a smaller footprint and have a lower throughput volume capability than the immunoassay laboratory instruments 220a and 220b.

The automated laboratory system 200 may include chemistry analyzer laboratory instruments 230a, 230b of a first type as well as chemistry analyzer laboratory instruments 240a, 240b and 240c of a second type. The automated laboratory system 200 may also include one or more post-analytics laboratory instruments 250, an automation component 260, and one or more pre-analytics laboratory instruments 270. The pre-analytics laboratory instrument 270 may comprise a loading area (not shown) for loading and identifying sample containers in the ALS 200, a centrifuge (not shown) for centrifuging sample containers and/or a decapper (not shown) for decapping sample containers.

Specifically, the automation component 260 includes a track, a belt and/or a tube carrier configured to move biological samples within the ALS 200. The automation component 260 is capable of transporting a container including a biological sample from one of the components 270, 210a, 210b, 220a, 220b, 230a, 230b, 240a, 240b, 240c, 250 of the ALS 200 to another component 270, 210a, 210b, 220a, 220b, 230a, 230b, 240a, 240b, 240c, 250 of the ALS 200. For instance, the automation component 260 is capable of transporting the container including the biological sample from the pre-analytics laboratory instruments 270 to one of the immunoassay laboratory instruments 210a, 210b, 220a, 220b.

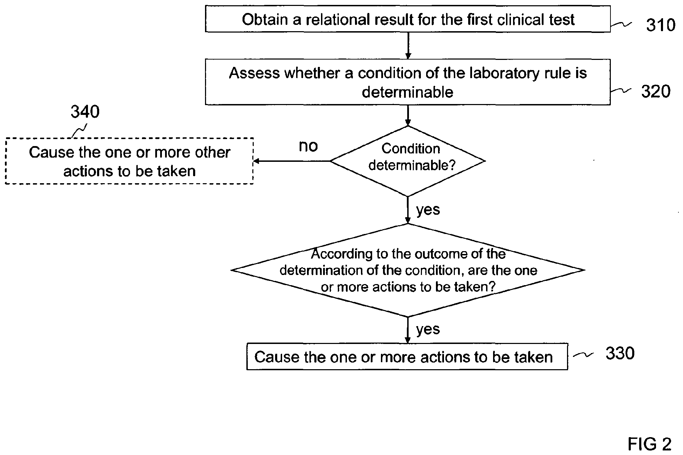

**Figure 2** shows a flow chart including steps of a method according to some embodiments. The method may be for controlling and/or operating a first laboratory instrument. The method may be for improving extraction of laboratory, clinical or diagnostic information from a clinical test.

At step 310, the method comprises obtaining, by a computing device (e.g., the computer 100) a relational result for a first clinical test. The first clinical test is carried out on a biological sample by a first laboratory instrument, the biological sample being in a sample container. For example, the first clinical test may be for quantitative enzymatic ultraviolet (UV) measurement of glucose. The first clinical test may be carried out on a 40 µL biological sample of cerebrospinal fluid (CSF) combined with about 4.5 mL of reagent. The reagent may include ISE Electrolyte buffer (about 1.27mL) and/or ISE Electrolyte Reference (about 3.23 mL). In addition, the biological sample may include Di- or tri-potassium (K2/K3) salts of ethylenediaminetetraacetic acid (EDTA). The measurement of glucose may be used in the diagnosis and treatment of a disorder or condition in a patient, such as diabetes mellitus, neonatal hypoglycemia or insulinoma. The first laboratory instrument may be a chemistry analyzer (e.g., one of the chemistry analyzer laboratory instruments 230a, 230b, 240a, 240b and 240c). The biological sample is in a sample container, e.g., a 10-15 ml test tube. The biological sample may be associated with a patient undergoing treatment at a medical facility, e.g., a hospital.

In some cases, at step 310, the computing device receives the relational result from the first laboratory instrument, said instrument being configured to assess whether the laboratory result of the first clinical test lies within the reliability range for the first clinical test carried out by the first laboratory instrument. If the laboratory result does not lie within the reliability range, the first laboratory instrument may be configured to construct the relational result. If the laboratory result is greater than the upper bound, U_{B}, the computing device constructs the following relational result >, U_{B}. If instead, the laboratory result is lower than the lower bound, L_{B}, the computing device constructs the following relational result <, L_{B}.

In some cases, at step 310, the computing device constructs the relational result by receiving the result from the first laboratory instrument and comparing the laboratory result with the upper bound and the lower bound of the reliability range for the first clinical test. In particular, the comparison aims at establishing whether the result provided by the first laboratory instrument lies within the reliability range or not. If the laboratory result is greater than the upper bound, U_{B}, the computing device constructs the following relational result >, U_{B}. If instead, the laboratory result is lower than the lower bound, L_{B}, the computing device constructs the following relational result <, L_{B}.

The relational result comprises first information and second information. The first information is comprised in the relational operator ">" or "<" and specifies that a laboratory result for the first clinical test is outside a reliability range for the first clinical test carried out by the laboratory instrument. Moreover, the relational operator ">" specifies that the laboratory result is greater than the upper bound U_{B} and the relational operator "<" specifies that the laboratory result is less that the lower bound L_{B}. In particular, the second information of the relational result specifies either the numerical value of the upper bound or the numerical value of the lower bound of the reliability range.

In a first example, the reliability range (e.g., analytical measuring range) of the clinical test to measure the concentration of a substance in CSF by using the chemistry analyzer may be 10 - 810 mg/dL. In particular, the laboratory result for that test may be less than 10 mg/dL. Accordingly, the laboratory instrument may indicate that the laboratory result is too low to be measured. Hence, the first information may specify that laboratory result is outside the reliability range, e.g., via a "<" relational operator. In the first example, 10 mg/dL is the lower bound of the reliability range and 810 mg/dL is the upper bound of the reliability range. Since the laboratory instrument indicates that the concentration of glucose in the CSF is too low to be measured and the reliability range is 10-810 mg/dL, the second information may specify "10", i.e., the lower bound of the reliability range. In the first example, the second information further include the units "mg/dL". Hence, according to the example, the relational result including first information and second information comprises "<", 10 and "mg/dL".

At step 320, the computing device assesses whether a condition of a laboratory rule is determinable by using the first information and the second information. In particular, at step 320, the computing device assesses whether the first and the second information allows for determining whether the condition is true or false.

For instance, in the first example, the laboratory rule may read as follows:
IF (*result of assessing glucose concentration < 18 mg*/*dL*) THEN (*indicate "High risk of bacterial meningitis")*
According to the laboratory rule, if the result of assessing the glucose concentration in the biological sample of CSF is less than 18 mg/dL, then the condition, "result of assessing glucose concentration < 18 mg/dL" is fulfilled.

In the first example, the first information, (comprised in the relational operator "<"), and the second information (i.e., the numerical value of the upper bound, 10, and the string "mg/dL" that specifies the unit of measure) specify that the laboratory result is less than 10 mg/dL. If the result is less than 10 mg/dL then the result must also be less than 18 mg/dL. Since it can be determined that the condition is fulfilled the condition is determinable.

The laboratory rule specifies one or more actions that are to be taken depending on the outcome of a determination of the condition. In the example, the action comprises indicating a high risk of bacterial meningitis. In this case, the action may include one or more of displaying an alert, transmitting an urgent message from the laboratory to the facility where the patient associated with the biological sample is being treated, and flagging the relational result as a result associated with high risk of bacterial meningitis. The alert and the message may indicate that there is a high risk that the patient has bacterial meningitis.

If the condition is determinable and, according to the outcome of the determination, the one or more actions are to be taken, the computing device causes the one or more actions to be taken (step 330). In particular, at step 330, the computing device provides the laboratory instrument with instructions to take at least one of the one or more actions and/or takes at least one of the one or more actions.

If the condition is not determinable and, according to the outcome of the determination, the one or more actions are not to be taken, the one or more actions specified in the rule are not taken.

In the first example, the condition is determined and is fulfilled and the computing device takes the actions specified by the rule. In particular, the computing device displays the alert on its video monitor, transmits the urgent message to the facility where the patient associated with the biological sample is being treated and flags the relational result as a result associated with high of bacterial meningitis.

Optionally, if the condition is not determinable, the computing device causes the one or more other actions to be taken (step 340). In particular, the one or more other actions may include instructing a second laboratory instrument to carry out a second clinical test. The second laboratory instrument may be configured to measure the parameter measured by the first laboratory instrument with a higher sensitivity, precision, specificity, linearity and/or accuracy than the first laboratory instrument. In some cases, the one or more other actions may include storing in a log file stored in its memory or in a database information specifying that the condition is not determinable.

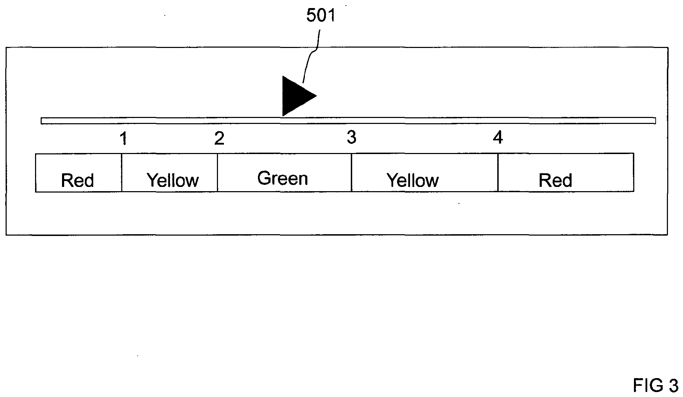

**Figure 3** shows an example of a display based on the laboratory result for a first clinical test and a reliability range for the first clinical test carried out by a laboratory instrument. In some cases, the one or more actions comprise displaying third information specifying whether, based on the first information and the second information, the laboratory result may belong to one or more of a critical range, a warning range and a normal range. In the example, the relational result obtained by the computing device reads >, 2.5. Moreover, in the example, a laboratory result, R, of the first clinical test belongs to:
∘ The critical (red) range if R < 1 or R > 4;
∘ The warning (yellow) range if 1 ≤ R > 2 or 3 < R ≤ 4; and
∘ The normal (green) range if 2 ≤ R ≤ 3.

In particular, the critical, warning and normal ranges above may be specified by one or more laboratory rules and the conditions "R < 1 or R > 4", "1 ≤ R > 2 or 3 < R ≤ 4" and "2 ≤ R ≤ 3" may be conditions of the one or more laboratory rules.

In the present example, the first information and the second information indicate that the laboratory result could be in the critical range, the warning range or the normal range. Therefore, in this example, the third information is included in the black arrow 501 indicating that it cannot be determined (i.e., at least one condition of the one or more laboratory rules is not determinable) whether the laboratory result lies in the normal range, the warning range or the critical range. The black arrow is pointing to the right (toward 4) to indicate that the relational result includes the ">" relational operator.

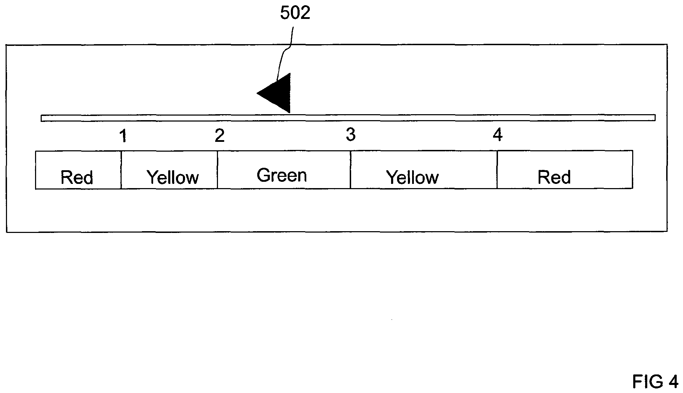

**Figure 4** shows another example of a display based on the laboratory result for a first clinical test and a reliability range for the first clinical test carried out by a laboratory instrument. This example differs from the previous example in that the relational result is <, 2.5. In this example, the first information and the second information indicate that the laboratory result could be in the critical range, the warning range or the normal range. Therefore, in this example, the third information is included in the black arrow 502 displayed in figure 4 that indicates that it cannot be determined (i.e., at least one condition of the one or more laboratory rules is not determinable) whether the laboratory result lies in the normal range, the warning range or the critical range. The black arrow is pointing to the left (toward 1) to indicate that the relational result includes the "<" relational operator.

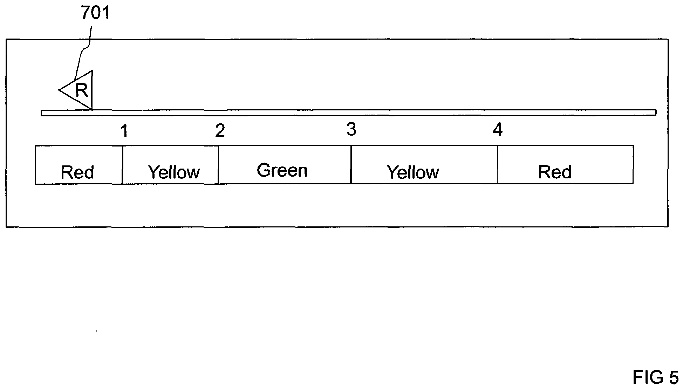

**Figure 5** shows another example of a display based on the laboratory result for a first clinical test and a reliability range for the first clinical test carried out by a laboratory instrument. This example differs from the previous example in that the relational result is <, 0.5. Hence, in this example, the first information and the second information indicate the laboratory result is in the critical range because the second information comprises a numerical value less than an upper boundary of the critical range (i.e., less than 1) and the first information indicates that the laboratory result is less than the numerical value of the second information. Hence, the third information specifies that the laboratory result belongs to the critical range and is included in the red arrow 701.

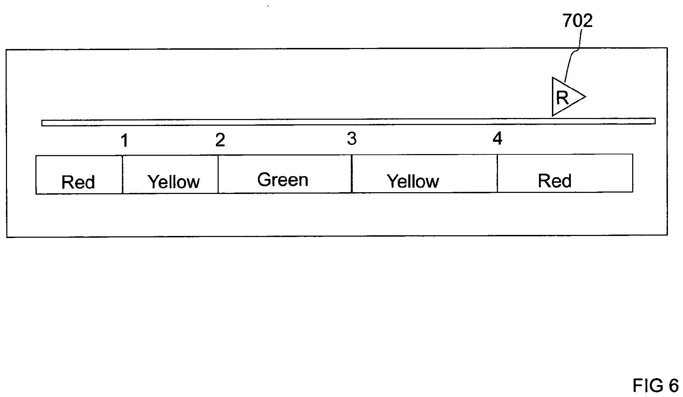

**Figure 6** shows yet another example of a display based on the laboratory result for a first clinical test and a reliability range for the first clinical test carried out by a laboratory instrument. This example differs from the previous example in that the relational result is >, 4.5. in this example, the first information and the second information indicate the laboratory result in is in the critical range because the second information comprises a numerical value greater than a lower boundary of the critical range (i.e., greater than 4) and the first information indicates that the laboratory result is greater than the numerical value of the second information. Therefore, the laboratory result is in the critical range and the red arrow 702 pointing right (i.e., away from the lower boundary of the critical range) is displayed as an indication thereof.

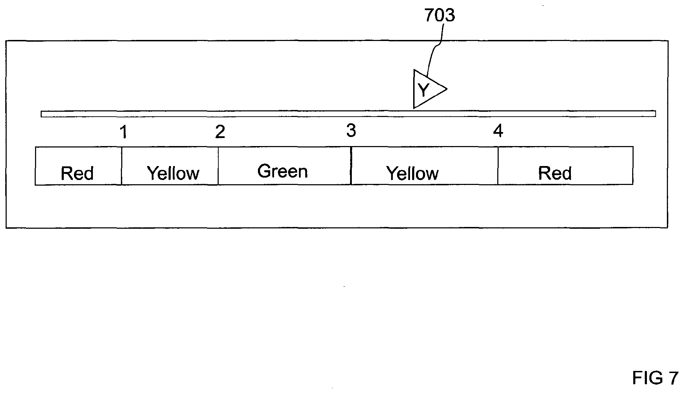

**Figure 7** shows yet another example of a display based on the laboratory result for a first clinical test and a reliability range for the first clinical test carried out by a laboratory instrument. This example differs from the previous example in that the relational result is >, 3.5. The first information and the second information indicate the laboratory result belongs to either the warning range or the critical range because the second information comprises a numerical value greater than a lower boundary of the warning range, i.e., a numerical value greater than 3, and the first information indicates that the laboratory result is greater than the numerical value of the second information. In this example, the third information specifies that the laboratory result may belong to the critical range or the warning range and is comprised in the yellow arrow 703 pointing right (away from the lower boundary of the warning range).

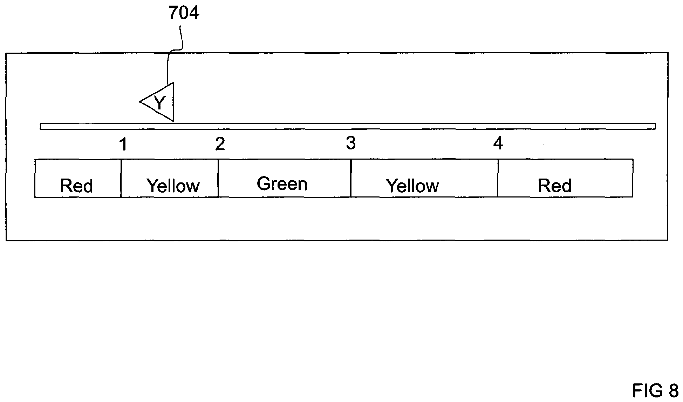

**Figure 8** shows yet another example of a display based on the laboratory result for a first clinical test and a reliability range for the first clinical test carried out by a laboratory instrument. This example differs from the previous example in that the relational result is >, 1.5. The first information and the second information indicate the laboratory result belongs to either the warning range or the critical range because the second information comprises a numerical value less than an upper boundary of the warning range, i.e., a numerical value less than 2, and the first information indicates that the laboratory result is less than the numerical value of the second information. Hence, the third information specifies that the laboratory result may belong to the critical range or the warning range and is comprised in the yellow arrow 704 pointing left (i.e., away from the upper boundary of the warning range).

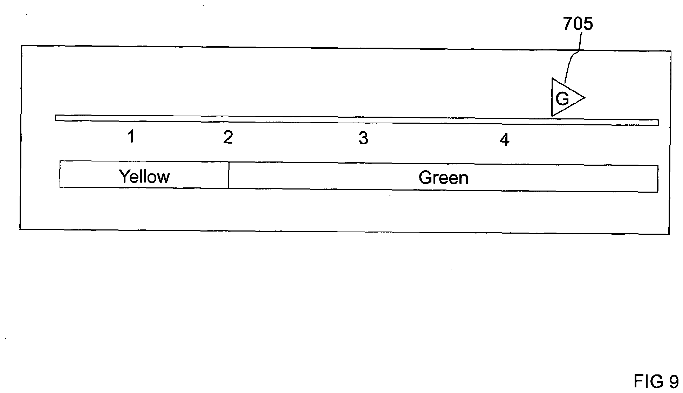

**Figure 9** shows yet another example of a display based on the laboratory result for a first clinical test and a reliability range for the first clinical test carried out by a laboratory instrument. In some cases, the one or more actions comprise displaying third information specifying whether, based on the first information and the second information, the laboratory result may belong to one or more of a warning range and a normal range. In the example, the relational result obtained by the computing device reads >, 4.5. Moreover, in the example, a laboratory result, R, of the first clinical test belongs to:
∘ The warning (yellow) range if R < 2; and
∘ The normal (green) range if R ≥ 2.

In the present example, the first information and the second information indicate that the laboratory result is in the normal range because the second information comprises a numerical value greater than a lower boundary of the normal range (i.e., greater than 2) and the first information indicates that the laboratory result is greater than the numerical value of the second information. Therefore, in this example, the third information specifies that the laboratory result belongs to the normal range and is included in the green arrow 705 pointing right (i.e., away from the lower boundary of the normal range).

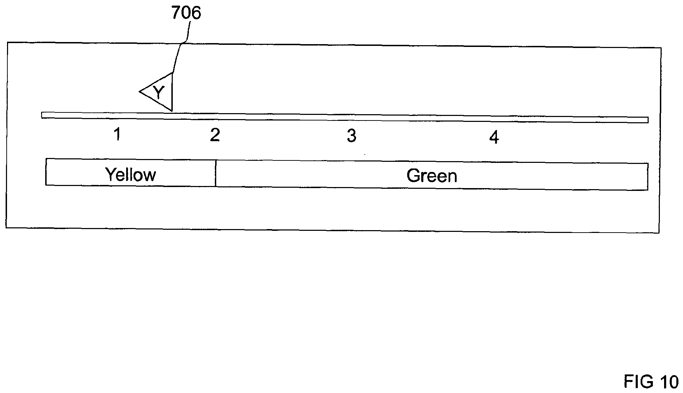

**Figure 10** shows yet another example of a display based on the laboratory result for a first clinical test and a reliability range for the first clinical test carried out by a laboratory instrument. This example differs from the previous example in that the relational result is <, 1.5. Hence, in this example, the first information and the second information indicate the laboratory result in is in the warning range because the second information comprises a numerical value less than an upper boundary of the warning range (i.e., less than 2) and the first information indicates that the laboratory result is less than the numerical value of the second information. Hence, the third information specifies that the laboratory result belongs to the warning range and is included in the yellow arrow 706 pointing left.

## Claims

1. A method comprising:
• obtaining, by a computing device, a relational result for a first clinical test, wherein:
- the first clinical test is carried out on a biological sample by a first laboratory instrument, the biological sample being in a sample container;
- the relational result comprises first information and second information, the first information specifying that a laboratory result for the first clinical test is outside a reliability range for the first clinical test carried out by the laboratory instrument and the second information specifying one or more of an upper bound and a lower bound of the reliability range;
• assessing, by the computing device, whether a condition of a laboratory rule is determinable by using the first information and the second information, wherein
- the laboratory rule specifies one or more actions that are to be taken depending on the outcome of a determination of the condition;
- at least one action of the one or more actions is to be taken on one or more of the biological sample, the sample container and the relational result and/or the one or more actions comprise displaying information about one or more of the biological sample, the sample container, the relational result and a patient;
and, if the condition is determinable and, according to the outcome of the determination of the condition, the one or more actions are to be taken:
• causing, by the computing device, the one or more actions to be taken.

2. The method of claim 1, wherein, if the condition is not determinable, the method further comprises causing one or more other actions to be taken.

3. The method of claim 2, wherein the one or more other actions comprise carrying out a second clinical test.

4. The method of claim 3, wherein the first clinical test comprises measuring a first clinical parameter using a first methodology and wherein the second clinical test comprises measuring the first clinical parameter using a second methodology.

5. The method of any one of the claims 2 to 4, wherein causing the one or more other actions to be taken comprises causing a second laboratory instrument to carry out the second clinical test.

6. The method of any one of claims 2 to 5, wherein the one or more other actions comprise notifying a user that the condition is not determinable and/or storing, in a computer-readable medium, information specifying that the condition is not determinable.

7. The method of any one of the preceding claims, wherein the one or more actions comprise re-running the first clinical test and/or carrying out a third clinical test.

8. The method of any one of the preceding claims, wherein the one or more actions comprise displaying third information, the third information specifying whether, based on the first information and the second information, the laboratory result may belong to one or more of a critical range, a warning range or a normal range wherein the critical range, the warning range and the normal are associated with a potential condition of a patient.

9. The method of any one of the preceding claims, wherein the second information specifies one or more of a unit of measure of the upper bound of the reliability range and a unit of measure of the lower bound of the reliability range.

10. The method of any one of the preceding claims, wherein causing the one or more actions to be taken comprises providing the laboratory instrument with instructions to take the one or more actions.

11. The method of any one of the preceding claims, wherein causing the one or more actions to be taken comprises taking the one or more actions.

12. A data processing system comprising means for carrying out the method according to any one of claims 1 to 11.

13. A laboratory instrument comprising the data processing system of claim 12, wherein the laboratory instrument is configured to receive the biological sample and/or to carry out the first clinical test.

14. A computer program comprising instructions which, when the program is executed by a computer, cause the computer to carry out the method of any one of claims 1 to 11.

15. A computer-readable medium comprising instructions which, when executed by a computer, cause the computer to carry out the method of any one of claims 1 to 11.
